# EUROPEAN PATENT APPLICATION

(11) **EP 4 198 132 A1**
(43) Date of publication of application: **21.06.2023**
(21) Application number: 21866964.6
(22) Date of filing: 30.06.2021
(51) Int. Cl.: C12N 15/77, C07K 14/195, C12P 13/14

(54) **RECOMBINANT MICROORGANISM FOR PRODUCING L-GLUTAMIC ACID, AND L-GLUTAMIC ACID PRODUCTION METHOD USING SAME**

(30) Priority: 09.09.2020 KR 20200115570
(71) Applicant: CJ Cheiljedang Corporation, Seoul 04560 (KR)
(72) Inventor: KWON, Nara, Seoul 04560 (KR); SONG, Gyuhyeon, Seoul 04560 (KR); LEE, Jin Nam, Seoul 04560 (KR); BONG, Hyun-Ju, Seoul 04560 (KR); SEO, Chang Il, Seoul 04560 (KR); LEE, Ah Reum, Seoul 04560 (KR)
(74) Representative: Regimbeau
(86) International application number: PCT/KR2021/008262
(87) International publication number: WO 2022/055094

(57) **Abstract**

The present disclosure relates to a L-glutamic acid-producing recombinant microorganism comprising a SbtA protein or a polynucleotide encoding the SbtA protein, and to a method for producing L-glutamic acid by using the same.

## Description

### [Technical Field]

The present disclosure relates to L-glutamic acid-producing microorganisms containing a SbtA protein or a polynucleotide encoding the SbtA protein and methods for producing L-glutamic acid by using the same.

### [Background Art]

L-Glutamic acid, which is a representative amino acid produced by fermentation, is one of the important amino acids that are widely used in the field of foods due to its unique, distinctive taste as well as in the field of medicinal products and the field of other animal feeds. There are known methods for producing L-glutamic acid by using microorganisms of the genus *Corynebacterium, Escherichia coli,* the genus *Bacillus, Streptomyces, Penicillium, Klebsiella, Erwinia,* or *Pantoea,* and the like (U.S. Patent Nos. 3,220,929 and 6,682,912).

Various studies are currently being conducted to develop microorganisms and fermentation process technology that produce L-glutamic acid with high efficiency. For example, target material-specific approaches, such as increasing the expression of genes encoding enzymes involved in amino acid biosynthesis or removing genes which are unnecessary for amino acid biosynthesis in the microorganisms of the genus *Corynebacterium,* are mainly used for an improvement in production yield of L-glutamic acid (U.S. Patent Nos. 9109242 B2 and 8030036 B2).

### [Disclosure]

### [Technical Problem]

The present inventors have conducted intensive studies to produce a high concentration of L-glutamic acid, and as a result, the present inventors have developed a microorganism of the genus *Corynebacterium* producing a high concentration of L-glutamic acid through the introduction of an exogenous protein thereinto, thus completing the present disclosure.

### [Technical Solution]

An object of the present disclosure is to provide a recombinant microorganism of the genus *Corynebacterium* producing L-glutamic acid, the recombinant microorganism containing a SbtA protein or a polynucleotide encoding the SbtA protein.

Another object of the present disclosure is to provide a method for producing L-glutamic acid, the method including culturing, in a medium, a recombinant microorganism of the genus *Corynebacterium* producing L-glutamic acid, the recombinant microorganism containing a SbtA protein or a polynucleotide encoding the SbtA protein.

### [Advantageous Effects]

The recombinant microorganism of the genus *Corynebacterium* producing L-glutamic acid and containing SbtA protein or a polynucleotide encoding SbtA protein can produce L-glutamic acid with high yield, and thus can be advantageously used in the industrial production of L-glutamic acid.

### [Brief Description of Drawing]

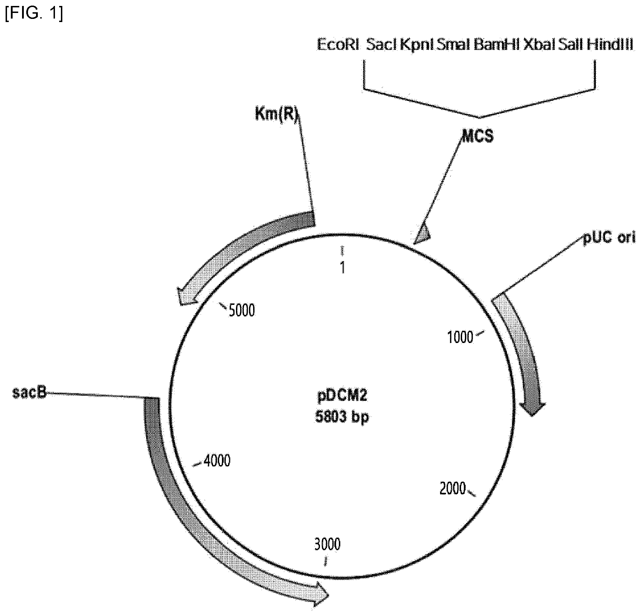

FIG. 1 is a schematic diagram of pDCM2 plasmid.

### [Best Mode]

The present disclosure will be specifically described as follows. Each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description below. Further, those skilled in the art will recognize or be able to ascertain, by using no more than routine experimentation, many equivalents to the specific embodiments of the present disclosure described herein. Such equivalents are intended to be encompassed by the present disclosure.

In accordance with an aspect of the present disclosure, there is provided a recombinant microorganism of the genus *Corynebacterium* producing L-glutamic acid, the recombinant microorganism containing a SbtA protein or a polynucleotide encoding the SbtA protein.

As used herein, the term "SbtA protein" refers to, as one of the bicarbonate transporters, one of the proteins that contribute to the acceleration of the processing of carbon dioxide (CO₂) by promoting the membrane passage of HCO₃⁻, a membrane impermeable bicarbonate, and are expressed for the capture and efficient use of carbon dioxide. Bicarbonate transporters include SbtA protein, BicA protein, and the like, which transport bicarbonate into cells, resulting in the accumulation of bicarbonate in the cells.

In the present disclosure, the SbtA protein may be a protein derived from a microorganism different from the microorganism of the present disclosure or may be different from a protein that is inherently present in the microorganism of the present disclosure.

In the present disclosure, the SbtA protein may be a protein derived from cyanobacteria, but is not limited thereto. Specifically, the SbtA protein may be a protein derived from a microorganism of the genus *Synechocystis,* and more specifically, it may be a protein derived from *Synechocystis* sp. PCC 6803 or *Synechocystis* sp. PCC 6714.

The SbtA protein of the present disclosure may contain an amino acid sequence having at least 90% sequence identity with the amino acid sequence of SEQ ID NO: 1. The amino acid sequence of SEQ ID NO: 1 may be obtained from the U.S. National Institutes of Health (NIH) GenBank, a known database. Specifically, the amino acid sequence having at least 90% identity with SEQ ID NO: 1 of the present disclosure may be an amino acid sequence having at least 91%, at least 92%, at least 93%, at least 94%, at least 95%, at least 96%, at least 96.26%, at least 97%, at least 97.5%, at least 97.7%, at least 97.8%, at least 98%, at least 98.5%, at least 98.7%, at least 98.8%, at least 99%, at least 99.5%, at least 99.7%, or at least 99.8%, but less than 100% homology or identity with SEQ ID NO: 1. For example, an amino acid sequence having at least 90% but less than 100% homology or identity with the amino acid sequence of SEQ ID NO: 1 may be the amino acid sequence of SEQ ID NO: 30. It is also obvious that when an amino acid sequence has such homology or identity and shows the equivalent or corresponding activity to the SbtA protein or the bicarbonate transporter activity, a protein having such an amino acid sequence also falls within the SbtA protein of the present disclosure even though the amino acid sequence has deletion, modification, conservative substitution, or addition in a part of the sequence thereof.

The SbtA protein of the present disclosure may have an amino acid sequence having at least 90% identity with the amino acids sequence of SEQ ID NO: 1. In addition, the SbtA protein of the present disclosure may have, consist of, or consist essentially of an amino acid sequence having at least 90% identity with the amino acid sequence of SEQ ID NO: 1. The SbtA protein of the present disclosure does not exclude an addition of a meaningless sequence upstream or downstream of the amino acid sequence of SEQ ID NO: 1 (i.e., an addition of a sequence that does not change functions of the protein to the N-terminus and/or C-terminus of the amino acid sequence), or a naturally occurring mutation, or a silent mutation thereof, or a conservative substitution.

In the SbtA protein, the term "conservative substitution" refers to a substitution of one amino acid with another amino acid having similar structural and/or chemical properties. Such an amino acid substitution may typically occur based on similarities in polarity, charge, solubility, hydrophobicity, hydrophilicity, and/or amphipathic nature of residues. Usually, the conservative substitution may have little or no effect on the activity of a protein or a polypeptide.

In the present disclosure, the polynucleotide encoding the SbtA protein may be the sbtA gene. In addition, the polynucleotide encoding the SbtA protein may be derived from cyanobacteria. Specifically, the polynucleotide encoding SbtA protein of the present disclosure may contain a nucleotide sequence encoding an amino acid sequence having at least 90% identity with the amino acid sequence of SEQ ID NO: 1. Specifically, the polynucleotide encoding the SbtA protein of the present disclosure may include the nucleotide sequence of SEQ ID NO: 2, the nucleotide sequence of SEQ ID NO: 31, or a nucleotide sequence having at least 90% identity therewith and encoding the amino acid sequence having at least 90% identity with the amino acid sequence of SEQ ID NO: 1, but is not limited thereto. The polynucleotide containing a nucleotide sequence encoding an amino acid sequence having at least 90% identity with the amino acid sequence of SEQ ID NO: 1 may be a polynucleotide having a nucleotide sequence encoding an amino acid sequence having at least 90% identity with the amino acid sequence of SEQ ID NO: 1 or a polynucleotide consisting of or consisting essentially of a nucleotide sequence encoding an amino acid sequence having at least 90% identity with the amino acid sequence of SEQ ID NO: 1.

As used herein, the term "polynucleotide" refers to a polymer of nucleotides in which nucleotide monomers are linked in a chain shape through covalent linkage and which is a DNA strand having a predetermined length or longer.

It is obvious that when a polynucleotide includes the nucleotide sequence of SEQ ID NO: 2, the nucleotide sequence of SEQ ID NO: 3, the nucleotide sequence of SEQ ID NO: 31, or a nucleotide sequence having at least 90% identity with these nucleotide sequences and encoding an amino acid sequence having at least 90% identity with the amino acid sequence of SEQ ID NO: 1, such polynucleotide falls within the polynucleotide of the present disclosure even though there is deletion, modification, conservative substitution, or addition in a part of each of the nucleotide sequences. In the polynucleotide of the present disclosure, the term "conservative substitution" refers to a substitution of one nucleotide with another nucleotide having similar structural and/or chemical properties.

The polynucleotide of the present disclosure may have various modifications in a coding region thereof within the scope in which the polynucleotide sequence is not changed, due to codon degeneracy or in consideration of the codons preferred by an organism in which the polynucleotide is to be expressed. Specifically, the polynucleotide of the present disclosure may be a polynucleotide containing the nucleotide sequence of SEQ ID NO: 2.

In addition, the polynucleotide of the present disclosure may include, without limitation, any polynucleotide sequence that can hybridize with a probe obtainable from the known sbtA gene sequence, for example, a sequence complementary to a part or the entirety of the nucleotide sequence, under stringent conditions, whereby the polynucleotide sequence can increase L-glutamic acid producing ability while not being a naturally present sequence in a microorganism for the introduction.

Alternatively, the polynucleotide of the present disclosure may include, without limitation, any sequence that can hybridize with a probe obtainable from a known gene sequence, for example, a sequence complementary to a part or the entirety of the polynucleotide sequence of the present disclosure, under stringent conditions. The term "stringent condition" refers to a condition that enables specific hybridization between polynucleotides. These conditions are specifically described in the literature (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof may include: a condition in which polynucleotides having higher homology or identity, for example, polynucleotides having at least 70%, at least 75%, at least 76%, at least 85%, at least 90%, at least 95%, at least 96%, at least 97%, at least 98%, or at least 99% homology or identity hybridize with each other, but polynucleotides having lower homology or identity do not hybridize with each other; or a condition in which washing is conducted once, specifically twice or three times, at a salt concentration and a temperature corresponding to 60°C, 1×SSC, 0.1% SDS, specifically 60°C, 0.1×SSC, 0.1% SDS, and more specifically 68°C, 0.1×SSC, 0.1% SDS, corresponding to washing conditions for typical Southern hybridization.

The hybridization requires two nucleic acids having complementary sequences although mismatches between nucleotides may be possible depending on the hybridization stringency. The term "complementary" is used to illustrate the relationship between nucleotides that can hybridize with each other. For example, as for DNA, adenine is complementary to thymine, and cytosine is complementary to guanine. Therefore, the polynucleotide of the present disclosure may also include not only substantially similar nucleic acid sequences but also isolated nucleic acid fragments complementary to the entire sequence.

Specifically, a polynucleotide having homology or identity with the polynucleotide of the present disclosure may be detected by using the above-described conditions and using hybridization conditions including a step of hybridization at Tₘ of 55°C. In addition, the Tₘ value may be 60°C, 63°C, or 65°C, but is not limited thereto, and may be appropriately regulated according to the purpose by a person skilled in the art.

An appropriate stringency for hybridizing the polynucleotides depends on the length of the polynucleotides and the degree of complementarity therebetween, and variables therefor are well known in the art (e.g., Sambrook et *al., supra*)*.*

As used herein, the term "homology" or "identity" refers to the extent of similarity between two given amino acid sequences or nucleotide sequences, and may be expressed as a percentage. The terms homology and identity may often be used interchangeably.

The sequence homology or identity of conserved polynucleotides or polypeptides may be determined using a standard alignment algorithm, and default gap penalties established by a program to be used may be used together. Substantially, homologous or identical sequences may generally hybridize with each other in the entirety or a part of each sequence under moderately or highly stringent conditions. It is obvious that the hybridization also includes a hybridization with a polynucleotide containing usual codons or codons considering codon degeneracy in the polynucleotide.

Whether any two polynucleotide or polypeptide sequences have homology, similarity, or identity may be determined using a known computer algorithm, such as the "FASTA" program, using default parameters as in Pearson et al., (1988) Proc. Natl. Acad. Sci. USA 85:2444. Alternatively, this may be determined using the Needleman-Wunsch algorithm (Needleman and Wunsch, 1970, J. Mol. Biol. 48:443-453), which is performed as in the Needleman program of the European Molecular Biology Open Software Suite (EMBOSS) package (Rice et al., 2000, Trends Genet. 16:276-277) (version 5.0.0 or versions thereafter) (including GCG program package (Devereux, J., et al., Nucleic Acids Research 12:387 (1984)), BLASTP, BLASTN, FASTA (Atschul, S. F., et al., J MOLEC BIOL 215:403 (1990); Guide to Huge Computers, Martin J. Bishop, ed., Academic Press, San Diego,1994, and CARILLO et al. (1988) SIAM J Applied Math 48:1073). For example, the homology, similarity, or identity may be determined using BLAST or ClustalW of the National Center for Biotechnology Information (NCBI).

The homology, similarity, or identity of polynucleotides or polypeptides may be determined by comparing sequence information through the GAP computer program, for example, Needleman et al., (1970), J Mol Biol. 48:443, as known in Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of symbols in the shorter of the two sequences. Default parameters for the GAP program may include: (1) a unary comparison matrix (containing a value of 1 for identity and 0 for non-identity) and the weighted comparison matrix of Gribskov et al. (1986) Nucl. Acids Res. 14:6745, as disclosed in Schwartz and Dayhoff, eds., Atlas Of Protein Sequence And Structure, National Biomedical Research Foundation, pp. 353-358 (1979) (or NCBI NUC4.4 EMOSSS version of substitution matrix); (2) a penalty of 3.0 for each gap and an additional penalty of 0.10 for each symbol in each gap (or a gap opening penalty of 10 and a gap extension penalty of 0.5); and (3) no penalty for an end gap.

The SbtA protein contained in the recombinant microorganism of the present disclosure may be expressed in the microorganism of the genus *Corynebacterium* by way of a conventional method known in the art.

As used herein, the term "expression" with respect to a protein refers to a state in which a target protein is introduced into a microorganism or a target protein is modified to be expressed in a microorganism. For the purpose of the present disclosure, the "target protein" may be the above-described SbtA protein.

As used herein, the term "introduction" with respect to a protein refers to an introduction of the activity of a specific protein into a microorganism free from the activity of the protein. This term may also be expressed as an enhancement of the activity of a specific protein in a microorganism free from the activity of the protein.

The introduction of the protein may be performed by introducing, into a host cell, an exogenous polynucleotide encoding a protein exhibiting the equivalent/similar activity to the protein, or a codon-optimized mutant-type polynucleotide thereof. The exogenous polynucleotide may be used without limitation to the origin or sequence thereof as long as the exogenous polynucleotide exhibits an equivalent/similar activity to the protein. In addition, for optimized transcription and translation of the introduced exogenous polynucleotide in the host cell, a codon thereof may be optimized and introduced into the host cell. The introduction may be performed by way of any known transformation method appropriately selected by a person skilled in the art, and through the expression of the introduced polynucleotide in the host cell, the protein is produced, and the activity thereof can be enhanced.

As used herein, the term "enhancement" of the activity of a polypeptide refers to an increase of the activity of a polypeptide compared with endogenous activity thereof. The enhancement may be used interchangeably with the terms activation, up-regulation, overexpression, increase, and the like. Herein, the activation, enhancement, up-regulation, overexpression, and increase may include all of: exhibiting an activity that has not been originally possessed; or exhibiting an activity improved compared with the endogenous activity or the activity before modification. The "endogenous activity" refers to the activity of a specific polypeptide originally possessed by a parent strain before modification or a non-modified microorganism when transformation occurs due to a genetic variation caused by native or artificial factors. The term may be used interchangeably with the "pre-modification activity". The "enhancement", "up-regulation", "overexpression", or "increase" of the activity of a polypeptide compared with the intrinsic activity thereof means an improvement in the activity and/or concentration (expression level) of a specific polypeptide originally possessed by a parent strain before modification or a non-modified microorganism.

The enhancement can be attained by the introduction of an exogenous polypeptide or through the enhancement of the inherent activity of the polypeptide and/or the concentration (expression level) thereof. The enhancement of the activity of the polypeptide can be identified by way of an increase in activity degree or expression level of the corresponding polypeptide or amount of a product produced from the corresponding polypeptide.

The enhancement of the activity of the polypeptide may be attained by applying various methods well known in the art, and is not limited as long as the activity of a target polypeptide is enhanced compared with a pre-modification microorganism. Specifically, genetic engineering and/or protein engineering well known to a person skilled in the art, which is a routine method of molecular biology, may be used, but is not limited thereto (e.g., Sitnicka et al., Functional Analysis of Genes. Advances in Cell Biology. 2010, Vol. 2. 1-16, Sambrook et al., Molecular Cloning 2012, *etc.*)*.*

Specifically, the enhancement of the activity of a polypeptide of the present disclosure may be:
1) an increase in intracellular copy number of the polynucleotide encoding the polypeptide;
2) a replacement of a gene expression control region on the chromosome encoding the polypeptide with a sequence with strong activity;
3) a modification of the nucleotide sequence encoding the initiation codon or 5'-UTR region of the gene transcript encoding the polypeptide;
4) a modification of the amino acid sequence of the polypeptide so as to enhance activity of the polypeptide;
5) a modification of the polynucleotide sequence encoding the polypeptide so as to enhance activity of the polypeptide (e.g., a modification of the polynucleotide sequence of the polypeptide gene so as to encode a polypeptide modified to enhance activity of the polypeptide);
6) an introduction of an exogenous polypeptide exhibiting activity of the polypeptide or an exogenous polypeptide encoding the same;
7) a codon optimization of the polynucleotide encoding the polypeptide;
8) a modification or chemical modification of an exposure site selected by analysis of a tertiary structure of the polypeptide; or
9) a combination of two or more selected from items 1) to 8), but is not particularly limited thereto.

More specifically, these are described as follows.

The increase in intracellular copy number of the polynucleotide encoding the polypeptide in item 1) may be attained by an introduction, into a host cell, of a vector to which the polynucleotide encoding the corresponding polypeptide is operably linked and which can replicate and function independently of a host. Alternatively, item 1) may be attained by an introduction of one copy or two or more copies of the polynucleotide encoding the corresponding polypeptide into the chromosome in a host cell. The introduction into the chromosome may be performed by way of an introduction, into a host cell, of a vector capable of inserting the polynucleotide into the chromosome in the host cell, but is not limited thereto.

The replacement of a gene expression control region on the chromosome encoding the polypeptide with a sequence with strong activity in item 2) may be, for example, a mutation occurring in the sequence through deletion, insertion, non-conservative or conservative substitution, or a combination thereof, so as to further enhance the activity of the expression control region, or a replacement with a sequence having stronger activity. The expression control region may include, but is not particularly limited to, a promoter, an operator sequence, a sequence encoding a ribosome binding site, a sequence controlling the termination of transcription and translation, and the like. For example, the replacement may be a replacement of the original promoter with a strong promoter, but is not limited thereto.

Known examples of the strong promoter may be CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, and the like, but are not limited thereto.

The modification of the nucleotide sequence encoding the initiation codon or 5'-UTR region of the gene transcript encoding the polypeptide in item 3) may be, for example, a substitution with a nucleotide sequence encoding an initiation codon enabling a higher expression rate of the polypeptide compared with the endogenous initiation codon, but is not limited thereto.

The modification of the amino acid sequence or the polynucleotide sequence in items 4) and 5) may be a mutation occurring through deletion, insertion, non-conservative or conservative substitution, or a combination thereof in the amino acid sequence of the polypeptide or the polynucleotide sequence encoding the polypeptide, so as to enhance activity of the polypeptide, or a replacement with an amino acid sequence or polynucleotide sequence modified to have stronger activity or an amino acid sequence or polynucleotide sequence modified to have increased activity, but is not limited thereto. Specifically, the replacement may be performed by inserting a polynucleotide into the chromosome by homologous recombination, but is not limited thereto. The vector used herein may further include a selection marker for checking the insertion of the chromosome. A selection marker is used for selection of cells transformed with the vector, i.e., to confirm whether the target nucleic acid molecule has been inserted, and markers which confer selectable phenotypes (e.g., drug resistance, auxotrophy, resistance to cytotoxic agents, expression of surface modified proteins, etc.) may be used. Under the circumstances where selective agents are treated, only the cells capable of expressing the selection markers can survive or express other phenotypic traits, thereby enabling easy selection of the transformed cells.

The introduction of an exogenous polypeptide exhibiting activity of the polypeptide in item 6) may be an introduction, into a host cell, of an exogenous polynucleotide encoding a polypeptide exhibiting the equivalent/similar activity to the polypeptide. The exogenous polynucleotide is not limited to the origin or sequence thereof as long as the exogenous polynucleotide exhibits the equivalent/similar activity to the polypeptide. The introduction may be performed by way of any known transformation method appropriately selected by a person skilled in the art, and through the expression of the introduced polynucleotide in the host cell, the polypeptide is produced, and the activity thereof can be enhanced.

The codon optimization of the polynucleotide encoding the polypeptide in item 7) may be a codon optimization of an endogenous polynucleotide so as to increase transcription or translation thereof in a host cell, or a codon optimization of an exogenous polynucleotide so as to make optimized transcription or translation thereof in a host cell.

The modification or chemical modification of an exposure site selected by analysis of a tertiary structure of the polypeptide in item 8) may be, for example, such that template protein candidates are determined according to the degree of sequence similarity by comparing the sequence information of a polypeptide to be analyzed with a database storing sequence information of base proteins, an exposure site to be modified or chemically modified is selected by identifying a structure on the basis of the candidates, and the exposure site is modified or chemically modified.

Such an enhancement of the activity of the polypeptide may mean that there is an increase in the activity or concentration (expression level) of the corresponding polypeptide compared with the activity or concentration of the polypeptide expressed in the wild-type or non-modified microorganism strains; or an increase in an amount of a product obtained from the corresponding polypeptide, without being limited thereto.

The modification of a part or the entirety of the polynucleotide in the microorganism of the present disclosure may be induced by: (a) genome editing adopting homologous recombination or engineered nuclease (e.g., CRISPR-Cas9) using a vector for chromosomal insertion in a microorganism and/or (b) the treatment with light, such as ultraviolet light and radiation, and/or chemicals, but is not limited thereto. The method of modifying a part or the entirety of the gene may include a method using DNA recombinant technology. For example, a nucleotide sequence or vector containing a nucleotide sequence homologous to a target gene may be introduced into the microorganism to bring about homologous recombination, resulting in a deletion in a part or the entirety of the gene. The nucleotide sequence or vector to be introduced may include a dominant selection marker, but is not limited thereto.

As used herein, the term "vector" refers to a DNA construct which contains a target polynucleotide sequence operably linked to an appropriate control sequence such that a target gene can be introduced into an appropriate host. The control sequence may include a promoter capable of initiating transcription, any operator sequence for controlling transcription, a sequence for encoding a suitable mRNA ribosomal binding site, and a sequence for controlling the termination of transcription and translation. The vector, after transformation into an appropriate host, can replicate or function independently of the host genome, or may be integrated into the genome itself. For example, a target polynucleotide in the chromosome may be replaced with a modified polynucleotide through a vector for chromosomal insertion in a cell. The insertion of the polynucleotide into the chromosome may be performed using any method known in the art, for example, homologous recombination, but is not limited thereto.

The vector of the present disclosure is not particularly limited, and any vector known in the art may be used. Examples of the vector commonly used may include native or recombinant plasmids, cosmids, viruses, and bacteriophages. For example, pWE15, M13, MBL3, MBL4, IXII, ASHII, APII, t10, t11, Charon4A, Charon21A, and the like may be used as phage vectors or cosmid vectors; and pBR-based, pUC-based, pBluescriptll-based, pGEM-based, pTZ-based, pCL-based, and pET-based vectors may be used as plasmid vectors. Specifically, pDZ, pACYC177, pACYC184, pCL, pECCG117, pUC19, pBR322, pMW118, or pCC1BAC vector may be used. More specifically, the vector used in the present disclosure may be pDCM2 (FIG. 1, SEQ ID NO: 32) constructed for insertion and replacement of a gene in the *Corynebacterium* chromosome, but is not particularly limited thereto, and a known expression vector may be used.

As used herein, the term "transformation" means that a vector containing a polynucleotide encoding a target protein is introduced into a host cell to express the protein encoded by the polynucleotide in the host cell. The transformed polynucleotide may include any polypeptide that can be expressed in a host cell, regardless of whether the polynucleotide is inserted and located into the chromosome or located outside of the chromosome of the host cell. In addition, the polynucleotide includes DNA and RNA encoding target proteins. The polynucleotide may be introduced in any form as long as it can be introduced into a host cell and expressed. For example, the polynucleotide may be introduced in the form of an expression cassette, which is a gene construct containing all factors required for self-expression. The expression cassette may usually include a promoter operably linked to the polynucleotide, a transcription termination signal, a ribosome binding site, and a translation terminal signal. The expression cassette may be in the form of an expression vector enabling self-replication. In addition, the polynucleotide may be introduced in the form as it is into the host cell and operably linked to the sequence required for expression in the host cell, but is not limited thereto.

As used herein, the term "operably linked" refers to a functional linkage between the gene sequence and a promoter sequence for initiating and mediating the transcription of the polynucleotide encoding the target protein of the present disclosure.

A method of transforming the vector of the present disclosure includes any method of introducing a nucleic acid into a cell, and any suitable standard technique known in the art may be selected and performed depending on the host cell. Examples of the technique may be electroporation, calcium phosphate (CaPO₄) precipitation, calcium chloride (CaCl₂) precipitation, micro-injection, a polyethyleneglycol (PEG) method, a DEAE-dextran method, a cationic liposome method, a lithium acetate-DMSO method, and the like, but are not limited thereto.

As used herein, the term "recombinant microorganism" includes all of microorganisms with artificial genetic modifications, and refers to a microorganism in which a particular mechanism is weakened or enhanced due to the insertion of an exogenous gene for expression of an exogenous protein or the enhancement or weakening of activity of the endogenous gene and which may be a microorganism including a genetic modification for production of a target protein or product.

For example, the recombinant microorganism of the present disclosure may be: a microorganism genetically modified through any one of SbtA protein or a polynucleotide encoding SbtA protein and a vector including the polynucleotide; a microorganism modified to express the protein or a polynucleotide encoding the protein; a recombinant microorganism expressing the protein or a polynucleotide encoding the same; or a recombinant microorganism having activity of the protein, but is not limited thereto.

In addition, the recombinant microorganism of the present disclosure may be a microorganism having an increased ability to produce L-glutamic acid by additionally enhancing the activity of some proteins in the L-glutamic acid biosynthesis pathway or additionally weakening the activity of some proteins in the L-glutamic acid degradation pathway.

Specifically, the microorganism of the present disclosure may be a microorganism with OdhA protein additionally weakened. More specifically, the microorganism of the present disclosure may be a microorganism with OdhA gene deleted. The sequences of the OdhA protein may be obtained from NCBI GenBank, a known database, and for example, may be GenBank Accession No. WP_060564343.1, but is not limited thereto, and may include those exhibiting the equivalent activity, without limitation.

The weakening of OdhA protein or the deletion of OdhA gene is merely one example, and without limitation thereto, the microorganism of the present disclosure may include various microorganisms wherein the activity of proteins in the L-glutamic acid biosynthesis pathway is enhanced or the activity of proteins in the L-glutamic acid degradation pathway is weakened.

As still another example, the recombinant microorganism of the present disclosure may use as a parent strain of a microorganism treated with a compound so as to enable the production of L-glutamic acid, and specifically, the compound may be *N*-methyl-*N*'-nitro-*N*-nitrosoguanidine (NTG), but is not limited thereto.

For the purpose of the present disclosure, the recombinant microorganism of the present disclosure refers to a microorganism with an improved ability to produce L-glutamic acid compared with a parent strain before modification or a non-modified microorganism. The "non-modified microorganism" does not exclude a strain containing a naturally occurring mutation in microorganisms, and may be a native microorganism itself, a wild-type microorganism itself, or a microorganism before the regulation of the expression level of a gene involved in the L-glutamic acid biosynthesis pathway, or a microorganism before the introduction of the sbtA gene, which is not endogenously present.

In the present disclosure, the parent strain may be a wild-type *Corynebacterium glutamicum* ATCC13869, *Corynebacterium glutamicum* with OdhA weakened in the *Corynebacterium glutamicum* ATCC13869, a strain with OdhA deleted in the *Corynebacterium glutamicum* ATCC13869, or *Corynebacterium glutamicum* BL2 (Accession No. KFCC-11074, Korean Patent No. 10-0292299) obtained by treating the *Corynebacterium glutamicum* KFCC 10656 strain with a mutagenic agent, such as *N*-methyl-*N*'-nitro-*N-*nitrosoguanidine (NTG).

As used herein, the term "weakening" of a polypeptide has a concept including all of the decrease or absence of activity compared with the inherent activity. The weakening may be used interchangeably with inactivation, deficiency, down-regulation, decrease, reduction, attenuation, or the like.

The weakening may also include: a case where the activity of the polypeptide itself is decreased or eliminated compared with the activity of the polypeptide itself possessed by the original microorganism due to the mutation or the like of the polynucleotide encoding the polypeptide; a case where the activity and/or concentration (expression level) of the entire polypeptides in the cell is lower compared with the native strain due to the inhibition of the expression of a gene of a polynucleotide encoding the polypeptide or the inhibition of the translation into the polypeptide; a case where the expression of the polynucleotide is not made; and/or a case where the polypeptide has no activity in spite of the expression of the polynucleotide. The "endogenous activity" refers to the activity of a specific polypeptide originally possessed by a parent strain before modification or a wild-type or non-modified microorganism when transformation occurs due to genetic variation caused by native or artificial factors. This term may be used interchangeably with the "activity before modification". The "weakening", "inactivation", "deficiency", "decrease", "down-regulation", "reduction", or "attenuation" of the activity of a polypeptide compared with the endogenous activity means that the activity of the polypeptide is lowered compared with the activity of a specific polypeptide originally possessed by a parent strain before modification or a non-modified microorganism.

The weakening of the activity of the polypeptide may be attained by any method known in the art, but is not limited thereto, and may be attained by applying various methods well known in the art (e.g., Nakashima N et al., Bacterial cellular engineering by genome editing and gene silencing. Int J Mol Sci. 2014;15(2):2773-2793, Sambrook et al., Molecular Cloning 2012, *etc.*)*.*

Specifically, the weakening of the polypeptide of the present disclosure may be:
1) a deletion of a part or the entirety of the gene encoding the polypeptide;
2) a modification of an expression control region (or expression control sequence) so as to reduce the expression of the gene encoding the polypeptide;
3) a modification of an amino acid sequence constituting the polypeptide so as to eliminate or weaken the activity of the polypeptide (*e.g.,* deletion/substitution/addition of at least one amino acid on the amino acid sequence);
4) a modification of the gene sequence encoding the polynucleotide so as to eliminate or weaken the activity of the polypeptide (*e.g.,* deletion/substitution/addition of at least one nucleotide on the nucleotide sequence of the polypeptide gene so as to encode the polypeptide modified to eliminate or weaken the activity of the polypeptide);
5) a modification of a nucleotide sequence encoding the initiation codon or 5'-UTR region of the gene transcript encoding the polypeptide;
6) an introduction of an antisense oligonucleotide (*e.g.,* antisense RNA) complementarily binding to the gene transcript encoding the polypeptide;
7) an addition of a sequence complementary to the Shine-Dalgarno sequence of the gene encoding the polypeptide upstream of the Shine-Dalgarno sequence so as to form a secondary structure that makes the attachment of ribosomes impossible;
8) an addition of a reverse transcription promoter to the 3' end of the open reading frame (ORF) of the gene sequence encoding the polypeptide (reverse transcription engineering, RTE); or
9) a combination of two or more selected from items 1) to 8), but is not particularly limited thereto.

For example, these are described as follows.

The deletion of a part or the entirety of the gene encoding the polypeptide in item 1) may be an elimination of the entirety of the polynucleotide encoding an endogenous target protein in the chromosome, a replacement with a polynucleotide with a deletion of some nucleotides, or a replacement with a marker gene.

The modification of an expression control region (or expression control sequence) in item 2) may be a mutation on the expression control region (or expression control sequence) through deletion, insertion, non-conservative or conservative substitution, or a combination thereof, or a replacement with a sequence having weaker activity. The expression control region includes a promoter, an operator sequence, a sequence for encoding a ribosomal binding site, and a sequence for controlling the termination of transcription and translation, but is not limited thereto.

The modification of the nucleotide sequence encoding the initiation codon or 5'-UTR region of the gene transcript encoding the polypeptide in item 5) may be, for example, a substitution with a nucleotide sequence encoding, rather than the endogenous initiation codon, another initiation codon having a lower expression rate of the polypeptide, but is not limited thereto.

The modification of the amino acid sequence or the polynucleotide sequence in items 3) and 4) may be a modification on the sequence through deletion, insertion, non-conservative or conservative substitution, or a combination thereof in the amino acid sequence of the polypeptide or the polynucleotide sequence encoding the polypeptide, so as to weaken the activity of the polypeptide, or a replacement with an amino acid sequence or polynucleotide sequence modified to have weaker activity or an amino acid sequence or polynucleotide sequence modified to have no activity, but is not limited thereto. For example, the expression of the gene may be inhibited or attenuated by introducing a mutation into the polynucleotide sequence to form a termination codon.

The introduction of an antisense oligonucleotide (*e.g.,* antisense RNA) complementarily binding to the gene transcript encoding the polypeptide in item 6) may be referred to, for example, the literature (Weintraub, H. et al., Antisense-RNA as a Genetics, Vol. 1(1) 1986).

The addition of a sequence complementary to the Shine-Dalgarno sequence of the gene encoding the polypeptide upstream of the Shine-Dalgarno sequence so as to form a secondary structure that makes the attachment of ribosomes impossible in item 7) may make mRNA translation impossible or reduce the rate thereof.

The addition of a reverse transcription promoter to the 3' end of the open reading frame (ORF) of the gene sequence encoding the polypeptide (reverse transcription engineering, RTE) in item 8) may make an antisense nucleotide complementary to the transcript of the gene encoding the polypeptide to thereby weaken the activity of the polypeptide.

The recombinant microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* and more specifically *Corynebacterium glutamicum,* but is not limited thereto.

The recombinant microorganism of the present disclosure may have an increased or improved ability to produce L-glutamic acid compared with non-modified microorganisms.

In accordance with another aspect of the present disclosure, there is provided a method for producing L-glutamic acid, the method including culturing a recombinant microorganism of the genus *Corynebacterium* producing L-glutamic acid, the recombinant microorganism including a SbtA protein or a polynucleotide encoding the SbtA protein.

The "SbtA protein", "polynucleotide encoding SbtA protein", and "recombinant microorganism" are as described above.

The SbtA protein may be derived from cyanobacteria, but is not limited thereto, which is as described above.

The SbtA protein may have at least 90% identity with SEQ ID NO: 1, but is not limited thereto, which is as described above.

The recombinant microorganism of the genus *Corynebacterium* may be *Corynebacterium glutamicum,* but is not limited thereto, which is as described above.

In the recombinant microorganism, OdhA may be additionally weakened or odhA may be deleted. In addition, the OdhA may contain the amino acid sequence of SEQ ID NO: 29, but is not limited thereto, which is as described above.

In addition, the recombinant microorganism may use as a parent strain of an NTG-treated microorganism, but is not limited thereto, which is as described above.

As used herein, the term "culture" refers to growing the microorganism in appropriately adjusted environment conditions. The culture procedure of the present disclosure may be performed according to appropriate media or culture conditions known in the art. This culture procedure may be easily adjusted and employed depending on the strain by a person skilled in the art. Specifically, the culture may be a batch type, a continuous type, and a fed-batch type of culture, but is not limited thereto.

As used herein, the "medium" refers to a mixture containing as main ingredients nutrient substances required for the culture of the microorganism, wherein the medium supplies nutrient substances, growth factors, and the like, including water that is essential for survival and growth. Specifically, as for the medium and other culture conditions used for culturing the microorganism of the present disclosure, any medium that is used for usual culture of microorganisms may be used without particular limitation. However, the microorganisms of the present disclosure may be cultured under aerobic conditions in a conventional medium containing appropriate carbon sources, nitrogen sources, phosphorus sources, inorganic compounds, amino acids, and/or vitamins, while the temperature, pH, and the like are adjusted. Specifically, the culture medium for the genus *Corynebacterium* may be found in the literature ("Manual of Methods for General Bacteriology" by the American Society for Bacteriology (Washington D.C., USA, 1981)).

In the present disclosure, the carbon sources may include: carbohydrates, such as glucose, saccharose, lactose, fructose, sucrose, and maltose; sugar alcohols, such as mannitol and sorbitol; organic acids, such as pyruvic acid, lactic acid, and citric acid; and amino acids, such as glutamic acid, methionine, and lysine. In addition, natural organic nutrient sources, such as starch hydrolysates, molasses, blackstrap molasses, rice bran, cassava, sugarcane bagasse, and corn steep liquor, may be used, and specifically, carbohydrates, such as glucose and sterile pretreated molasses (i.e., molasses converted to reduced sugars) may be used, and appropriate amounts of other carbon sources may be used without limitation. These carbon sources may be used alone or in a combination of two or more, but are not limited thereto.

As for the nitrogen sources, inorganic nitrogen sources, such as ammonia, ammonium sulfate, ammonium chloride, ammonium acetate, ammonium phosphate, ammonium carbonate, and ammonium nitrate; amino acids, such as glutamic acid, methionine, and glutamine; and organic nitrogen sources, such as peptone, NZ-amine, meat extracts, yeast extracts, malt extracts, corn steep liquor, casein hydrolysates, fishes or their decomposition products, defatted soybean cake or its decomposition products, and the like may be used. These nitrogen sources may be used alone or in a combination of two or more, but are not limited thereto.

The phosphorus sources may include potassium phosphate monobasic, potassium phosphate dibasic, and corresponding sodium-containing salts. As for inorganic compounds, sodium chloride, calcium chloride, iron chloride, magnesium sulfate, iron sulfate, manganese sulfate, calcium carbonate, and the like may be used, and additionally, amino acids, vitamins, and/or suitable precursors may be included. These constituent ingredients or precursors may be added to the medium in a batch or continuous manner. However, the medium of the present disclosure is not limited thereto.

The pH of the medium may be adjusted by adding compounds, such as ammonium hydroxide, potassium hydroxide, ammonia, phosphoric acid, and sulfuric acid, to the medium in a suitable manner during the culture of the microorganism. In addition, a antifoaming agent, such as fatty acid polyglycol ester, may be added to suppress foam formation. In addition, oxygen or oxygen-containing gas may be injected into the medium to maintain the aerobic state of the medium, or, to maintain the anaerobic or non-aerobic state of the medium, nitrogen, hydrogen, or carbon dioxide gas may be injected, or no gas may be injected, but this is not limited thereto.

The temperature of the medium may be 20°C to 45°C, and specifically 25°C to 40°C, but is not limited thereto. The culture period may continue until a desired amount of a useful substance produced can be obtained, and may be specifically 10 to 160 hours, but is not limited thereto.

L-Glutamic acid produced by the culture may be released into the medium or may remain in cells without being released.

The method for producing L-glutamic acid of the present disclosure may further include a step of preparing a recombinant microorganism of the genus *Corynebacterium* producing L-glutamic acid before the culturing step, or a step of preparing a medium for culturing the recombinant microorganism.

Furthermore, the method for producing L-glutamic acid of the present disclosure may further include a step of recovering L-glutamic acid from the recombinant microorganism or the medium, after the culturing step.

The method of recovering L-glutamic acid may be collecting desired L-glutamic acid by using an appropriate method known in the art according to the method for culturing a microorganism of the present disclosure, for example, a batch type, a continuous type, and a fed-batch type of culture method. For example, centrifugation, filtration, anion-exchange chromatography, crystallization, HPLC, and the like may be used, and desired L-glutamic acid can be recovered from the medium or microorganism by using a suitable method known in the art.

In addition, the recovering step may include a purification process, and may be performed using a suitable method known in the art.

The recovered L-glutamic acid may be in a purified form, or may be a microorganism fermentation liquid containing L-glutamic acid, but is not limited thereto.

In accordance with still another aspect of the present disclosure, there is provided a composition for producing L-glutamic acid, the composition containing: a recombinant microorganism of the genus *Corynebacterium* producing L-glutamic acid, the recombinant microorganism including a SbtA protein or a polynucleotide encoding the SbtA protein; or a medium having the recombinant microorganism cultured therein.

The "SbtA protein", "polynucleotide encoding SbtA protein", "recombinant microorganism", and "medium" are as described above.

The composition of the present disclosure may further contain any suitable excipient that is usually used in the composition for producing L-glutamic acid, and examples of the excipient may be a preserving agent, a wetting agent, a dispersing agent, a suspending agent, a buffer, a stabilizing agent, or an isotonic agent, but are not limited thereto.

### [Mode for Invention]

Hereinafter, the present disclosure will be described in detail with reference to exemplary embodiments. However, these exemplary embodiments are merely preferable embodiments for illustrating the present disclosure, and therefore are not intended to limit the scope of right of the present disclosure. Meanwhile, the technical matters not described herein can be sufficiently understood and easily implemented by a person skilled in the art or technical fields similar to that of the present disclosure.

### Example 1: Construction of plasmid

A plasmid (pDCM2, FIG. 1, SEQ ID NO: 32) for the insertion and replacement of a gene in the *Corynebacterium* chromosome was designed, and the plasmid was synthesized using the Gene-synthesis service of BIONICS Co., Ltd. The plasmid was designed to include restriction enzymes facilitating the use in cloning with reference to a paper associated with the generally known sacB system (Gene, 145 (1994) 69-73). The pDCM2 plasmid thus synthesized had the following characteristics.
1) The plasmid has a replication origin that works only in *E. coli,* and thus the self-replication thereof is possible in *E. coli,* but the self-replication thereof is impossible in *Corynebacterium.*
2) The plasmid has the kanamycin resistance gene as a selection marker.
3) The plasmid has the Levan sucrose gene (sacB) as a secondary positive selection marker.
4) No genetic information derived from the pDCM2 plasmid is left in the finally constructed strain.

### Example 2: Construction of bicarbonate transporter expression vectors derived from cyanobacteria

To investigate the improvement of L-glutamic acid producing ability when sbtA and bicA genes encoding bicarbonate (HCO₃⁻) transporters derived from cyanobacteria, specifically *Synechocystis* sp. PCC6803, are introduced into strains, expression vectors for sbtA or bicA were first constructed.

To insert the two genes into *Corynebacterium glutamicum,* a codon-optimized sequence (SEQ ID NO: 2) of *Synechocystis* sp. PCC6803-derived sbtA (SEQ ID NO: 3) for *Corynebacterium glutamicum* and a codon-optimized sequence (SEQ ID NO: 5) of *Synechocystis* sp. PCC6803-derived bicA (SEQ ID NO: 6) for *Corynebacterium glutamicum* were synthesized.

The pDCM2 vector was used for insertion of the two genes, and CJ7 synthetic promoter (Korean Patent No. 10-0620092 and WO 2006-065095) was used as an expression promoter.

Specifically, the pDCM2-ΔCglE0286::CJ7-sbtA vector and the pDCM2-ΔCglE0085::CJ7-bicA vector were, respectively, constructed by using the sites of BBD29_01410 (hereafter, called CglE0286) and BBD29_00440 (hereafter, called CglE0085) in the transposases or integrases of *Corynebacterium glutamicum,* as sites of chromosomal homologous recombination.

Specifically, gene fragments at upstream and downstream regions of CglE0286 where chromosomal homologous recombination occurs were obtained through PCR using *Corynebacterium glutamicum* ATCC13869 chromosomal DNA as a template along with a primer set of SEQ ID NO: 7 and SEQ ID NO: 8, SEQ ID NO: 9 and SEQ ID NO: 10. Solg^{™} Pfu-X DNA polymerase was used as the polymerase, and PCR amplification conditions were: denaturation at 95°C for 5 minutes; 30 cycles of denaturation at 95°C for 30 seconds, annealing at 58°C for 30 seconds, and polymerization at 72°C for 60 seconds; and then polymerization at 72°C for 5 minutes.

The amplified upstream and downstream regions of CglE0286, and the pDCM2 vector for chromosomal transformation, which was cleaved with EcoRI and Sall restriction enzymes, were cloned using the Gibson assembly method (DG Gibson et al., NATURE METHODS, Vol. 6 No. 5, MAY 2009, NEBuilder HiFi DNA Assembly Master Mix) to thereby obtain a recombinant plasmid, which was then named pDCM2-ΔCglE0286. The cloning was performed by mixing the Gibson assembly reagent and each of the gene fragments in calculated numbers of moles, followed by preservation at 50°C for 1 hour.

PCR was performed using the synthesized CJ7-sbtA of SEQ ID NO: 11 as a template along with the primers of SEQ ID NO: 12 and SEQ ID NO: 13, and the CJ7-sbtA gene fragment was obtained through PCR. Solg^{™} Pfu-X DNA polymerase was used as the polymerase, and PCR amplification conditions were: denaturation at 95°C for 5 minutes; 30 cycles of denaturation at 95°C for 30 seconds, annealing at 58°C for 30 seconds, and polymerization at 72°C for 60 seconds; and then polymerization at 72°C for 5 minutes.

The amplified CJ7-sbtA, and the pDCM2-ΔCglE0286 vector for chromosomal transformation, which was cleaved with Scal restriction enzyme, were cloned using the Gibson assembly method to thereby obtain a recombinant plasmid, which was then named pDCM2-ΔCglE0286::CJ7-sbtA. The cloning was performed by mixing the Gibson assembly reagent and each of the gene fragments in calculated numbers of moles, followed by preservation at 50°C for 1 hour.

In addition, gene fragments of the upstream and downstream regions of CglE0085 where chromosomal homologous recombination occurs were obtained through PCR using *Corynebacterium glutamicum* ATCC13869 chromosomal DNA as a template along with a primer set of SEQ ID NO: 14 and SEQ ID NO: 15, SEQ ID NO: 16 and SEQ ID NO: 17. Solg^{™} Pfu-X DNA polymerase was used as the polymerase, and PCR amplification conditions were: denaturation at 95°C for 5 minutes; 30 cycles of denaturation at 95°C for 30 seconds, annealing at 58°C for 30 seconds, and polymerization at 72°C for 60 seconds; and then polymerization at 72°C for 5 minutes.

The amplified upstream and downstream regions of CglE0085, and the pDCM2 vector for chromosomal transformation, which was cleaved with EcoRI and Sall restriction enzymes, were cloned using the Gibson assembly method to thereby obtain a recombinant plasmid, which was then named pDCM2-ΔCglE0085. The cloning was performed by mixing the Gibson assembly reagent and each of the gene fragments in calculated numbers of moles, followed by preservation at 50°C for 1 hour.

PCR was performed using the synthesized CJ7-bicA of SEQ ID NO: 18 as a template along with the primers of SEQ ID NO: 12 and SEQ ID NO: 13 to thereby obtain the CJ7-bicA gene fragment. Solg^{™} Pfu-X DNA polymerase was used as the polymerase, and PCR amplification conditions were: denaturation at 95°C for 5 minutes; 30 cycles of denaturation at 95°C for 30 seconds, annealing at 58°C for 30 seconds, and polymerization at 72°C for 60 seconds; and then polymerization at 72°C for 5 minutes.

The amplified CJ7-bicA, and the pDCM2-ΔCglE0085 vector for chromosomal transformation, which was cleaved with Scal restriction enzyme, were cloned using the Gibson assembly method to thereby obtain a recombinant plasmid, which was then named pDCM2-ΔCglE0085::CJ7-bicA. The cloning was performed by mixing the Gibson assembly reagent and each of the gene fragments in calculated numbers of moles, followed by preservation at 50°C for 1 hour.

The primer sequences used herein are shown in Table 1 below.

**TABLE 1**

| Sequence number | Name | Sequence |
|---|---|---|
| 7 | CglE0286 _up_F | AACGACGGCCAGTGAATTCCGGACGTTTACGCTGA T |
| 8 | CglE0286 _up_R | AGTACTAAACCGGAAGGGCCTGTAAAGGCC |
| 9 | CglE0286 down F | CCTTCCGGTTTAGTACTAAACAGGAAGAGCCCCTTT A |
| 10 | CglE0286 down R | TGCATGCCTGCAGGTCGACCGCATGTGGTGGCAA AA |
| 12 | CJ7-sbtA F | GGCCCTTCCGGTTTAGT |
| 13 | CJ7-sbtA R | GGCTCTTCCTGTTTAGT |
| 14 | CglE0085 _up_F | AACGACGGCCAGTGAATTCGCTCGAATGCCTGACT GACA |
| 15 | CglE0085 _up_R | GTTTAGTACTAAACCGGAAGGGCCAAAGGACGACT TCACGGTTA |
| 16 | CglE0085 _down_F | CCGGTTTAGTACTAAACAGGAAGAGCCATTGAGGA TGCGAAACTGT |
| 17 | CglE0085 _down_R | TGCATGCCTGCAGGTCGACGTAATCGAATCACCGG CCAG |

The pDCM2-ΔCglE0286::CJ7-sbtA vector and the pDCM2-ΔCglE0085::CJ7-bicA vector thus constructed were introduced into strains in the following examples.

### Example 3: Preparation of L-glutamic acid-producing strain derived from wild-type Corynebacterium glutamicum and preparation of strains with bicarbonate transporters introduced thereinto

### Example 3-1: Preparation of Corynebacterium glutamicum derived from wild-type Corynebacterium glutamicum and having L-glutamic acid producing ability

To prepare a strain derived from *Corynebacterium glutamicum* ATCC13869 and having L-glutamic acid producing ability, the *Corynebacterium glutamicum* ATCC13869ΔodhA strain with a deletion of odhA gene was prepared based on the prior art (Appl Environ Microbiol. 2007 Feb;73(4):1308-19. Epub 2006 Dec 8.)

Specifically, for the deletion of odhA, the upstream and downstream regions of the odhA gene were obtained through PCR using *Corynebacterium glutamicum* ATCC13869 chromosomal DNA as a template along with a primer set of SEQ ID NO: 19 and SEQ ID NO: 20, SEQ ID NO: 21 and SEQ ID NO: 22. Solg^{™} Pfu-X DNA polymerase was used as the polymerase, and PCR amplification conditions were: denaturation at 95°C for 5 minutes; 30 cycles of denaturation at 95°C for 30 seconds, annealing at 58°C for 30 seconds, and polymerization at 72°C for 60 seconds; and then polymerization at 72°C for 5 minutes.

The amplified upstream and downstream regions of odhA, and the pDCM2 vector for chromosomal transformation, which was cleaved with Smal restriction enzyme, were cloned using the Gibson assembly method to thereby obtain a recombinant plasmid, which was then named pDCM2-ΔodhA. The cloning was performed by mixing the Gibson assembly reagent and each of the gene fragments in calculated numbers of moles, followed by preservation at 50°C for 1 hour.

The constructed pDCM2-ΔodhA vector was transformed into the *Corynebacterium glutamicum* ATCC13869 strain by way of electroporation, followed by secondary crossover to thereby obtain a strain with a deletion of odhA gene on the chromosome. The gene deletion was identified through PCR using SEQ ID NO: 23 and SEQ ID NO: 24 and genome sequencing, and the prepared strain was named ATCC13869ΔodhA.

The primer sequences used herein are shown in Table 2 below.

**TABLE 2**

| Sequence number | Name | Sequence |
|---|---|---|
| 19 | odhA_up_F | TGAATTCGAGCTCGGTACCCTTGAACGGAATT GGGTGG |
| 20 | odhA_up_R | CCCAGGTGGCATCGGTACCTTCACCCAGCGC CACGCAG |
| 21 | odhA_down_F | CGCTGGGTGAAGGTACCGATGCCACCTGGGT TGGTCAAG |
| 22 | odhA_down_R | GTCGACTCTAGAGGATCCCCGGACAAGGAAT GGAGAGA |
| 23 | odhA_del_F | CTTACCGTTGTTGCCCTT |
| 24 | odhA_del_R | CTCCTTCACCCACATCATT |

### Example 3-2: Preparation of strains with bicarbonate transporters introduced thereinto derived from Corynebacterium glutamicum and having L-glutamic acid producing ability

The effect of the introduction of a bicarbonate transporter on the L-glutamic acid producing ability was investigated by introducing the pDCM2-ΔCglE0286::CJ7-sbtA vector and the pDCM2-ΔCglE0085::CJ7-bicA vector constructed in Example 2 into the ATCC13869ΔodhA strain prepared in Example 3-1.

Specifically, the constructed pDCM2-ΔCglE0286::CJ7-sbtA vector in Example 2 was transformed into the *Corynebacterium glutamicum* ATCC13869ΔodhA strain by way of electroporation, followed by secondary crossover to thereby obtain a strain with an insertion of CJ7-sbtA at the site of CglE0286 on the chromosome.

The corresponding gene manipulation was identified through PCR using SEQ ID NO: 25 and SEQ ID NO: 26 capable of amplifying the external sites of the upstream and downstream regions of the homologous recombination into which CJ7-sbtA gene was introduced, and genome sequencing, and the prepared strain was named CA02-1474.

In addition, the pDCM2-ΔCglE0085::CJ7-bicA vector in Example 2 was transformed into the *Corynebacterium glutamicum* ATCC13869ΔodhA strain by way of electroporation, followed by secondary crossover to thereby obtain a strain with an insertion of CJ7-bicA at the site of CglE0085 on the chromosome.

The corresponding gene manipulation was identified through PCR using SEQ ID NO: 27 and SEQ ID NO: 28 capable of amplifying the external sites of the upstream and downstream regions of homologous recombination into which the CJ7-bicA gene is introduced, and genome sequencing, and the prepared strain was named CA02-1475.

The primer sequences used herein are shown in Table 3 below.

**TABLE 3**

| Sequence number | Name | Sequence |
|---|---|---|
| 25 | CJ7-sbtA_confirm_F | CAAGCAGCAAGTGCCACAC |
| 26 | CJ7-sbtA_confirm_R | CCAACTGCTCCGACGAAG |
| 27 | CJ7-bicA_confirm_F | ATTCCCAAAGGTGCCAGC |
| 28 | CJ7-bicA_confirm_R | CAGTGCGCAAAAGGCCTC |

The CA02-1474 and CA02-1475 strains prepared as above were cultured by the following method so as to investigate the L-glutamic acid producing ability thereof, while the ATCC13869ΔodhA strain was used as a control.

Each strain was inoculated into a plate medium composed of a seed medium, and cultured at 30°C for 20 hours. Then, one loop of the strain was inoculated into a 250 mL corner-baffle flask containing 25 mL of the production medium as below, and cultured with shaking at 30°C at 200 rpm for 40 hours. Upon the completion of the culture, the amount of L-glutamic acid produced was measured by high-performance liquid chromatography (HPLC), and the measurement results are shown in Table 4.

### <Seed medium>

1% glucose, 0.5% beef extract, 1% polypeptone, 0.25% sodium chloride, 0.5% yeast extract, 2% agar, 0.2% urea, pH 7.2

### <Production medium>

6% raw sugar, 5% calcium carbonate, 2.25% ammonium sulfate, 0.1% potassium monophosphate, 0.04% magnesium sulfate, 10 mg/L iron sulfate, 0.2 mg/L thiamine hydrochloride, 50 µg/L biotin

**TABLE 4**

| Strain name | L-Glutamic acid concentration (g/L) | L-Glutamic acid concentration increase (%) |
|---|---|---|
| ATCC13869 ΔodhA | 1.9 | - |
| CA02-1474 | 2.9 | 52.6% |
| CA02-1475 | 2.1 | 10.5% |

As shown in Table 4, the concentration of L-glutamic acid was significantly increased in CA02-1474 strain having sbtA gene introduced thereinto compared with the ATCC13869ΔodhA gene or the CA02-1475 strain having the bicA gene introduced thereinto.

CA02-1474 was deposited with the Korea Microorganism Conservation Center, a depositary authority under the Budapest Treaty, on July 28, 2020, and was assigned accession number KCCM12775P.

### Example 4: Preparation of Corynebacterium glutamicum strain with bicarbonate transporter introduced thereinto derived from N-methyl-N'-nitro-N-nitrosoguanidine (NTG) mutant and having L-glutamic acid producing ability

To investigate whether the gene showed the equivalent effect even in a strain derived from an NTG mutant *Corynebacterium* sp., and having increased L-glutamic acid producing ability as well, in addition to the strain derived from the wild-type *Corynebacterium* sp., the gene was introduced into the KFCC11074 strain (Korean Patent No. 10-0292299), known as an L-glutamic acid-producing NTG mutant.

The pDCM2-ΔCglE0286::CJ7-sbtA vector in Example 2 was transformed into the KFCC11074 strain by way of electroporation, followed by secondary crossover to thereby obtain a strain with an insertion of the gene on the chromosome. The corresponding gene manipulation was identified through PCR using SEQ ID NO: 25 and SEQ ID NO: 26 and genome sequencing, and the strain was named CA02-1476.

The prepared CA02-1476 and the *Corynebacterium glutamicum* KFCC11074 strain were subjected to a fermentation titer test according to the following method.

Each strain was inoculated into a plate medium composed of a seed medium, and cultured at 30°C for 20 hours. Then, one loop of the strain was inoculated into a 250 mL corner-baffle flask containing 25 mL of a production medium, and cultured with shaking at 30°C at 200 rpm for 40 hours. Upon the completion of the culture, the amount of L-glutamic acid produced was measured by high-performance liquid chromatography (HPLC), and the measurement results are shown in Table 5.

### <Seed medium>

1% Glucose, 0.5% beef extract, 1% polypeptone, 0.25% sodium chloride, 0.5% yeast extract, 2% agar, 0.2% urea, pH 7.2

### <Production medium>

6% raw sugar, 5% calcium carbonate, 2.25% ammonium sulfate, 0.1% potassium monophosphate, 0.04% magnesium sulfate, 10 mg/L iron sulfate, 0.2 mg/L thiamine hydrochloride, 500 µg/L biotin

**TABLE 5**

| Strain name | L-Glutamic acid concentration (g/L) | L-Glutamic acid concentration increase (%) |
|---|---|---|
| KFCC11074 | 6.9 | - |
| CA02-1476 | 9.3 | 34.8% |

As shown in Table 5, the CA02-1476 strain increased the concentration of L-glutamic acid by 34.8% compared with the KFCC11074 strain.

From the above description, a person skilled in the art to which the present disclosure pertains will be able to understand that the present disclosure may be embodied in other specific forms without departing from the technical spirit or essential characteristics thereof. In this regard, the exemplary embodiments disclosed herein are only for illustrative purposes and should not be construed as limiting the scope of the present disclosure. The scope of the present disclosure should be understood such that all changes or modifications derived from the definitions and scopes of the claims and their equivalents fall within the scope of the disclosure.

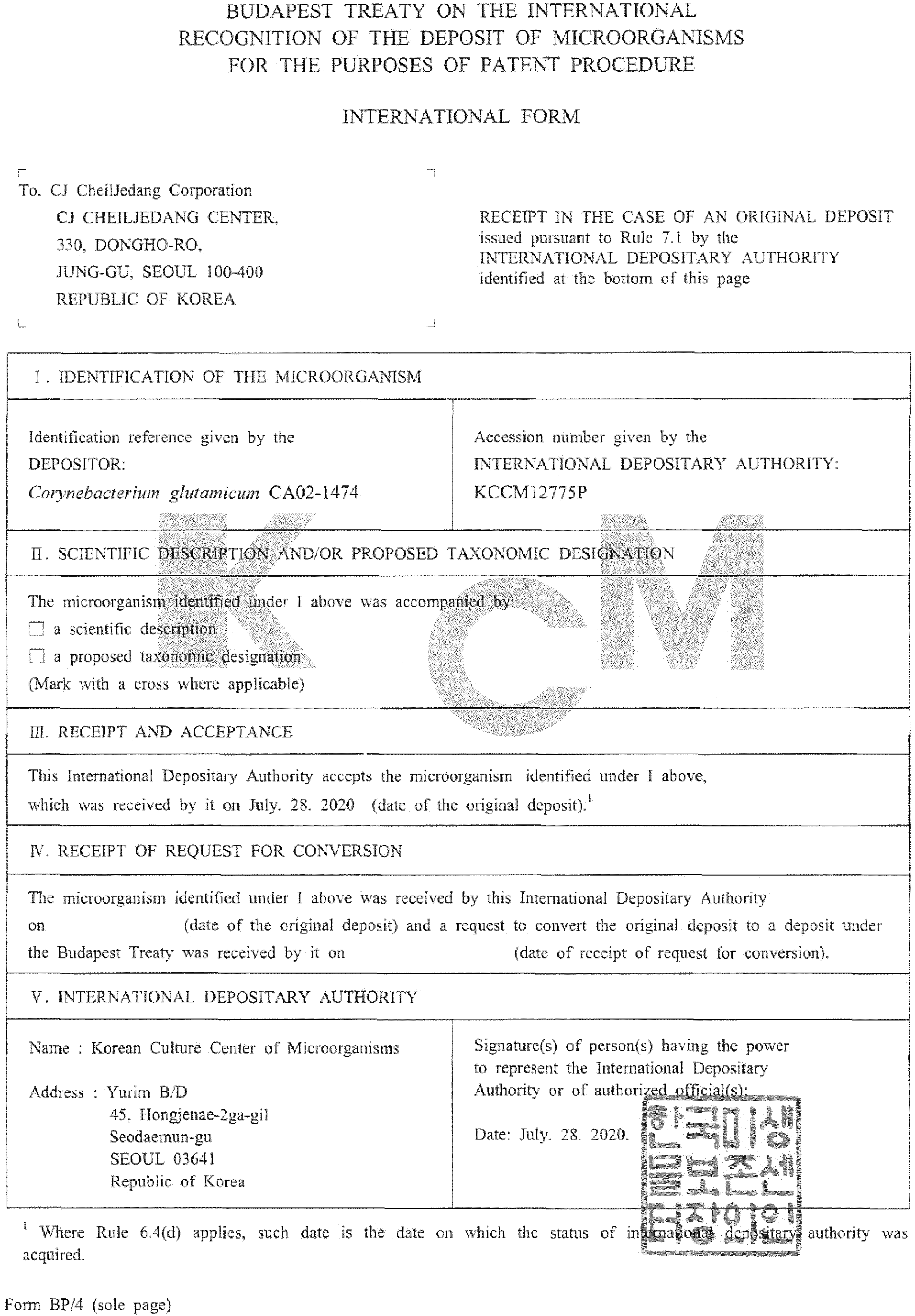
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CA02-1474 | Accession number given by the
INTERNATIONAL DEPOSITARY AUTHORITY:
KCCM12775P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on July. 28. 2020 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: July. 28. 2020.
한국미생
물보존센
터장인

¹ Where Rule 6.4(d) applies, such date is the date on which the status of international depositary authority was
acquired.

Form BP/4 (sole page)

## Claims

1. A recombinant microorganism of the genus *Corynebacterium* producing L-glutamic acid, the recombinant microorganism comprising a SbtA protein or a polynucleotide encoding the SbtA protein.

2. The recombinant microorganism of claim 1, wherein the SbtA protein is derived from cyanobacteria.

3. The recombinant microorganism of claim 1, wherein the SbtA protein contains the amino acid sequence of SEQ ID NO: 1 or an amino acid sequence having at least 90% sequence identity therewith.

4. The recombinant microorganism of claim 1, wherein the recombinant microorganism of the genus *Corynebacterium* is *Corynebacterium glutamicum.*

5. The recombinant microorganism of claim 1, in which activity of OdhA protein is further weakened.

6. A method for producing L-glutamic acid, the method comprising culturing, in a medium, a recombinant microorganism of the genus *Corynebacterium* producing L-glutamic acid, the recombinant microorganism comprising an SbtA protein or a polynucleotide encoding the SbtA protein.

7. The method of claim 6, wherein the SbtA protein is derived from cyanobacteria.

8. The method of claim 6, wherein the SbtA protein contains the amino acid sequence of SEQ ID NO: 1 or an amino acid sequence having at least 90% sequence identity therewith.

9. The method of claim 6, wherein the recombinant microorganism of the genus *Corynebacterium* is *Corynebacterium glutamicum.*

10. The method of claim 6, further comprising recovering L-glutamic acid from the recombinant microorganism or the medium.

11. The method of claim 6, wherein activity of OdhA protein is further weakened.
